# EUROPEAN PATENT SPECIFICATION

(11) **EP 0 947 201 B1**
(45) Date of publication and mention of the grant of the patent: **28.06.2006**
(21) Application number: 99101642.9
(22) Date of filing: 04.02.1999
(51) Int. Cl.: A61K 47/48, A61K 9/16, A61K 38/17

(54) **Pharmaceutical composition of hedgehog proteins and use thereof**
Pharmazeutische Zusammensetzungen von Hedgehog-Proteinen und deren Verwendung
Compositions pharmaceutiques contenant des protéines Hedgehog, et leur utilisation

(30) Priority: 04.02.1998 EP 98101893; 12.03.1998 EP 98104416
(43) Date of publication of application: 06.10.1999
(73) Proprietor: Curis, Inc., Cambridge, MA 02138 (US)
(72) Inventor: Lang, Kurt, 82377 Penzberg (DE); Papadimitriou, Apollon, 83673 Bichl (DE)
(74) Representative: Horner, Martin Grenville

(56) References cited:
- WO-A-90/05522
- WO-A-90/08551
- WO-A-95/18635
- WO-A-95/18856
- KIKUCHI A ET AL: "Pulsed dextran release from calcium-alginate gel beads" JOURNAL OF CONTROLLED RELEASE, vol. 47, no. 1, 7 July 1997, page 21-29 XP004091190
- ROBINSON C J: "Neurotrophic factors - novel therapeutics" TRENDS IN BIOTECHNOLOGY, vol. 14, no. 12, December 1996, page 451-452 XP004071034
- DOWNS E.C. ET AL: "Calcium alginate beads as a slow-release system for delivering angiogenic molecules in vivo and in vitro" J. CELL. PHYSIOL., 1992, VOL. 152, NO. 2, PAGE(S) 422-429, XP002072808
- GRAY, C. J. ET AL: "Retention of insulin in alginate gel beads" BIOTECHNOL. BIOENG., 1988, VOL. 31, NO. 6, PAGES 607-612, XP002072809
- Y. YANG ET AL.: "Relationship between dose, distance and time in Sonic Hedgehog-mediated regulation of anteroposterior polarity in the chick limb" DEVELOPMENT, vol. 124, 1997, pages 4393-4404, XP002072810
- A. LÓPEZ-MARTÍNEZ ET AL: "Limb-patterning activity and restreicted posterior localization of the amino-terminal product of Sonic hedgehog cleavage" CURRENT BIOLOGY, vol. 5, no. 7, 1995, pages 791-796, XP002072811
- E. MARTÍ ET AL.: "Requirement of 19K form of Sonic hedgehog for induction of distinct ventral cell types in CNS explants" NATURE, vol. 375, 25 May 1995, pages 322-325, XP002072812 LONDON, GB
- M.J. BITGOOD ET AL.: "Sertoli cell signaling by Desert hedgehog regulates the male germline" CURRENT BIOLOGY, vol. 6, no. 3, 1996, pages 298-304, XP002072813
- N. KINTO ET AL.: "Fibroblasts expressing Sonic hedgehog induce osteoblast differentiation and ectopic bone formation" FEBS LETTERS., vol. 404, 1997, pages 319-323, XP002072814 AMSTERDAM NL
- ILLUM L ET AL: "HYALURONIC ACID ESTER MICROSPHERES AS A NASAL DELIVERY SYSTEM FOR INSULIN" JOURNAL OF CONTROLLED RELEASE, vol. 29, no. 1/02, 1 February 1994, pages 133-141, XP000433658

## Description

The invention concerns a pharmaceutical composition of hedgehog proteins and its use in particular for the local release of these proteins on bones and cartilage.

Hedgehog (hh) proteins are understood as a family of secreted signal proteins which are responsible for the formation of numerous structures in embryogenesis (J.C. Smith, Cell 76 (1994) 193 - 196, N. Perrimon, Cell 80 (1995) 517 - 520, C. Chiang et al., Nature 83 (1996) 407, M.J. Bitgood et al., Curr. Biol. 6 (1996) 296, A. Vortkamp et al., Science 273 (1996) 613, C.J. Lai et al., Development 121 (1995) 2349). During its biosynthesis a 20 kD N-terminal domain and a 25 kD C-terminal domain are obtained after cleavage of the signal sequence and autocatalytic cleavage. The N-terminal domain is modified in its natural form with cholesterol (J.A. Porter et al., Science 274 (1996) 255 - 259). In higher life-forms the hh family is composed of at least three members i.e. sonic, indian and desert hh (Shh, Ihh, Dhh; M. Fietz et al., Development (Suppl.) (1994) 43 - 51). Differences in the activity of hedgehog proteins that were produced recombinantly were observed after production in prokaryotes and eukaryotes (M. Hynes et al., Neuron 15 (1995) 35 - 44 and T. Nakamura et al., Biochem. Biophys. Res. Comm. 237 (1997) 465 - 469.

Hynes et al. compare the activity of hh in the supernatant of transformed human embryonic kidney 293 cells (eukaryotic hh) with hh produced from E. coli and find a four-fold higher activity of hh from the supernatants of the kidney cell line. A potential additional accessory factor which is only expressed in eukaryotic cells, a post-translational modification, a different N-terminus since the hh isolated from E. coli contains 50 % of a hh which carries two additional N-terminal amino acids (Gly-Ser) or is shortened by 5 - 6 amino acids, or a higher state of aggregation (e.g. by binding to nickel agarose beads) have been discussed to be the reason for this increased activity.

Nakamura et al. compare the activity of shh in the supernatant of transformed chicken embryo fibroblasts with an shh fusion protein isolated from E. coli which still has an N-terminal polyhistidine part. The shh in the supernatant of the fibroblasts has a seven-fold higher activity than the purified E. coli protein with regard to stimulation of alkaline phosphatase (AP) in C3H10T ½ cells. Molecules such as bone morphogenetic proteins (BMPs) have been discussed as the reason for the increased activity which are only present in the supernatant of eukaryotic cells and cause the stronger induction of AP.

Kinto et al., FEBS Letters, 404 (1997) 319 - 323 describe that fibroblasts which secrete hh induce ectopic bone formation in an i.m. implantation on collagen. Hedgehog proteins therefore have an osteoinductive activity.

A process for the production of delivery systems for proteins with delayed release using alginate is known from WO 90/08551. In this process a two-phase system is formed in which the first phase contains a high concentration of the protein (saturated solution) and the second phase contains alginate. However, such a phase separation is difficult and complicated to carry out when producing pharmaceutical compositions in large amounts.

A pulsatile release of dextran from calcium-alginate complexes is known from Kikuchi, A. et al., J. Controlled Release 47 (1997) 21 - 29. However, the coupling of hedgehog proteins to such complexes is not described by Kikuchi.

Robinson, C.J., et al. describe in Trends in Biotechnology 14 (1996) 451 - 452 the intraventricular implantation of alginate microspheres as a method for the local application of NGF or NGF-secreting cells. However an application for hedgehog proteins is not described.

Downs, E.C. et al. describe in J. Cell. Physiol. 152 (1992) 422-429 the application of calcium alginate spheres as a delivery system for angiogenesis factors. However, the use of this process or this delivery system for hedgehog proteins is not described.

Crey, C.J. and J. Dowsett describe in Biotechnol. Bioeng. 31 (1988) 607 - 612 the use of calcium/zinc spheres as a delivery system for insulin. However, the use of this process for the production of delivery systems for hedgehog proteins is not known from this.

From Yang et al., Development 124 (1997) 4393 - 4404 it is known that high local hedgehog concentrations must be maintained over a period of at least 16 h at the site of action in the body of a pharmaceutically effective in vivo activity. The carrier system described by Yang et al. like the hedgehog-loaded chromatography medium Affigel CM, the Ni-agarose described by Marti et al. in Nature 375 (1995) 322 - 325 or the Affigel Blue used by Lopez-Martinez et al. in Curr. Biol. 5 (1995) 791 - 796 or the heparin-agarose particles that were used therein are unsuitable for a pharmaceutical application since they are immunogenic and can cause inflammatory reactions.

### International patent application number PCT/SE95/00011 (WO 95/18635) describes factors, such as GH, IGF-I, IGF-II and EGF, which can be delivered using a low molecular weight hyaluronic acid composition. However, an application for hedgehog proteins is not described.

The inventors found that the biocompatible and biodegradable carrier collagen described by Kinto et al. for hh-expressing cells is also unsuitable for an optimal local pharmaceutical application of hedgehog proteins as long as these hedgehog proteins bind to the carrier only via ionic interactions. It was found that when collagen carriers are loaded with hedgehog proteins under physiological conditions (pH ca. 7 and in weak acids (up to pH 4.5)) the majority of the applied hedgehog protein is released within minutes from the matrix. According to the inventors' findings, this insufficient binding is due to the lack of sufficient ionic interactions between the hedgehog protein and the carrier. In the case of loading under acidic conditions (below pH 4.5), a large amount of the applied hedgehog protein is denatured and irreversibly bound to the carrier.

Hence the object of the invention is to provide a pharmaceutical composition of a hedgehog protein with a biocompatible carrier wherein the carrier binds the hedgehog protein in its active, folded structure and can release it in vivo in its active form in a delayed manner. Such formulations are especially suitable for repairing bone and cartilage defects, but can also be used to repair neuronal defects or for a systemic delivery.

The object is achieved by a pharmaceutical composition according to claim 1 of a hedgehog protein which is characterized in that the hedgehog protein is bound to a hydrophilic carrier which is biocompatible wherein the carrier is a polymer which
- binds the hedgehog protein as a negatively-charged carrier as a result of ionic interactions,
- does not denature the hedgehog protein when it binds to the carrier,
- the carrier contains at least 0.1 to 1, preferably 0.1 to 2 negatively-charged residues per monomer under neutral conditions,
- the charge is mediated in the form of acidic groups such as e.g. sulfate, carboxyl or phosphate groups,
- and the average molecular weight of the carrier is at least 50,000 Da.

It has surprisingly turned out that hedgehog proteins can be released reversibly from a carrier in vivo in an active form and in a delayed manner without causing homogeneous and/or inflammatory reactions in vivo if they are bound to a negatively-charged soluble or insoluble polymer matrix.

Preferably a hydrophilic carrier matrix is used and particularly preferably a soluble or insoluble organic hydrophilic carrier matrix. The carrier matrix is particularly preferably composed of an anionic poly-saccharide such as preferably hyaluronic acid (as well as chemically cross-linked forms thereof), chondroitin sulfate, polyvinyl sulfate, keratin sulfate, dextran sulfate, pectin, carrageenans and other hydrocolloids, sulfated alginate, dermatan sulfate, alginate, preferably calcium alginate or a combination of at least two such anionic polysaccharides or a combination of these charged polysaccharides with other polymers, such as in particular collagen, in which the percentage by weight of the charged polysaccharides is 10-50 %. Insoluble matrix in the sense of the invention means that the matrix is essentially not decomposed or does not visibly dissolve in buffered solution in vitro within 10 - 20 hours at room temperature. In this connection it is particularly preferable that the carrier used according to the invention contains less than 50 %, preferably less than 20 % and particularly preferably essentially no amount of a neutral polysaccharide. Hyaluronic acid with a molecular weight of at least 10⁶ Dalton, in particular with a molecular weight of 4 x 10⁶ Dalton is particularly suitable as a carrier matrix.

In a further embodiment it has turned out that hydrophilic carriers based on inorganic insoluble phosphate such as hydroxylapatite or tricalcium phosphate are also suitable according to the invention as an insoluble carrier matrix.

A delayed release according to the invention is understood as a release of the hedgehog protein in a pharmacologically effective dose over a defined period of at least 14 hours. A pharmacological effect is understood as a neurological effect on nerve cells, a chondrogenesis and/or chondrogenesis induction and preferably osteogenesis and/or osteoinduction as described by Kinto et al., FEBS Letters, 404 (1997) 319-323 for bone induction, by Miao et al. in J. Neurosci. 17 (1997) 5891-5899 for the effect on nerve cells and by Stott et al. in J. Cell. Sci. 110 (1997) 2691-2701 for cartilage cell induction.

An enzymatically degradable carrier is preferably used as the carrier which can be degraded by secreted enzymes (e.g. proteases) from the cells on which the local in vivo application is carried out. However, the half-life of the carrier should be at least 12 h, but can be several weeks. If the carrier is composed of a polysaccharide, this carrier is preferably degraded by glycosidases and by hydrolases that are present in the cell and secreted. Such a biodegradability of the carrier is, however, not necessary in every case. If the release is carried out to treat osteoporosis or neuronal diseases, a biodegradability is not necessary. However, such carriers are preferably poorly soluble under physiological conditions and consequently are absorbed by the body over a relative long period (several weeks to months).

Solutions of the hedgehog proteins in high concentrations are necessary in order to produce carrier matrices which are coated with hedgehog proteins in such a manner that they exhibit an adequate pharmaceutical efficacy when applied locally. It has turned out that carriers coated with hedgehog protein that can be used pharmaceutically should preferably contain a concentration of the hedgehog protein of 1 to 5 mg/ml, preferably 3 mg/ml carrier, or more. Carriers are particularly preferred which contain hedgehog proteins at a concentration of 10 mg/ml or more. Hedgehog proteins are intrinsically poorly soluble. However, it has surprisingly turned out that the solubility of the hedgehog proteins increases drastically in solutions which contain arginine or argininium ions (preferably argininium sulfate). Hence, a further subject matter of the invention are aqueous solutions of hedgehog proteins at a concentration of 3 mg/ml and more which contain arginine and argininium ions and are preferably buffered. A further subject matter of the invention is a process for the production of a carrier matrix coated with hedgehog protein which is characterized in that the carrier matrix is incubated with a hedgehog protein solution at a concentration of 3 mg/ml which contains arginine or argininium ions and the carrier matrix coated in this manner is isolated.

Such solutions are suitable for producing carrier matrices which contain hedgehog proteins in pharmaceutically effective concentrations and are suitable for a pharmaceutical application.

Activity within the sense of the invention is understood as the activity of alkaline phosphatase (stimulation of the expression of alkaline phosphatase) which the polypeptide can induce in mammalian cells (activity in the alkaline phosphatase assay). For this a mouse mesenchymal cell line is cultured in a medium which contains fetal calf serum. Subsequently sterile-filtered sample is added, the cells are lysed after ca. 5 days and alkaline phosphatase is determined in the cell lysate by means of the cleavage of a chromogenic substrate (pNP, p-nitrophenol) (J. Asahina, Exp. Cell. Res. 222 (1996) 38 - 47 and T. Nakamura (1997)).

A hedgehog protein is understood by the invention as a secreted signal protein which is responsible for the formation of numerous structures in embryogenesis. Sonic, indian or desert hh are particularly preferably used (Fietz M., et al. Development (Suppl.) (1994) 43-51). A hh protein with a sequence as described in the EMBL database under the No. L38518 is preferably used. Proteins of the hedgehog family exhibit a pronounced homology in their amino acid sequence which is why it is also preferable to express those nucleic acids which code for hedgehog proteins which are 80 % or more homologous with the above-mentioned sequence of sonic hedgehog protein.

The human sonic hedgehog precursor protein is composed of the amino acids 1 - 462 of the sequence described in the EMBL database under No. L38518. The amino acids 1 - 23 represent the signal peptide, the amino acids 24 - 197 represent the mature signal domain, the amino acids 32 - 197 represent the signal domain shortened by 8 amino acids and the amino acids 198 - 462 represent the auto-processing domain after autoproteolytic cleavage.

The pharmaceutical composition according to claim 1 preferably contains an additional polymer which essentially acts as a supporting structural substance, which preferably also has an adhesion function for cells but does not bind on hedgehog proteins based on ionic interactions. Preferably, this substance is a biodegradable protein or a hydrolytic degradation product which may be used, for example, in the form of intact protein fibers as solubilized protein or as partially hydrolyzed protein. Such a supporting structural substance is preferably collagen, gelatin, elastin, or fibrin. The supporting structural substance is preferably present in a lower amount than the described hydrophilic biocompatible carrier according to the invention. The proportion of the supporting structural substance is therefore preferably 30%, or less preferably 10% or less. However, the supporting structural substance can also be present in excess relative to the hydrophilic carrier. It only needs to be ensured in this connection that the amount of hydrophilic carrier in the pharmaceutical composition according to the invention be sufficiently high to ensure that a therapeutically effective amount of the hedgehog protein is bound to the hydrophilic carrier. It is therefore preferred to use at least a 5-fold excess of the hydrophilic carrier referred to the hedgehog protein. In addition, the binding of the hedgehog protein to the carrier for the preparation of the pharmaceutical composition should be effected at a pH of 4.5 or greater. As pointed out above, it has been found that hedgehog proteins at a pH below 4.5, denature and bind irreversibly to proteinous carrier-like collagen. Therefore the binding of the hedgehog protein to the carrier should be effected in the neutral pH range.

Carriers which contain, apart from the hydrophilic carrier, further supporting structural compounds are, for example, protein / polysaccharide complexes. Preferred complexes are described in U.S. Patent 4,614,794.

The pharmaceutical composition is prepared by incubating the hedgehog protein with the hydrophilic carrier at a pH of 4.5 or greater, preferably a pH within the neutral range (pH 6 to 8), whereby the binding of the hedgehog protein to the carrier is effected. The incubation is carried out preferably in a buffered solution. When using as the carrier a matrix which additionally contains a biodegradable protein such as collagen, incubation at a pH of 4.5 or greater will ensure the prevention of irreversible binding of hedgehog protein (which is denatured at lower pH values) to the biodegradable protein. It has been found that under these conditions, no binding, or only a negligible binding, of the hedgehog protein to the biodegradable protein occurs as long as the hedgehog protein does not contain a hydrophobic modification, and thus the biodegradable protein merely acts as a supporting structural substance.

Hydrophobically modified (lipophilised) hedgehog proteins are hedgehog proteins which show in relation to unmodified hh proteins (e.g., recombinantly produced in prokaryotes) an increase in surface hydrophobicity. The degree of lipophilisation of a protein is measured by integration in a lipid layer according to Haque, Z., et al., J. Agric. Food Chem. 30 (1982) 481. Such lipophilised hh proteins bind according to the invention to the hydrophilic carrier in the same manner as non-lipophilised hh protein does, but they bind, in addition, to the biodegradable protein via hydrophobic interactions.

For the production of the pharmaceutical composition it is additionally preferable to add auxiliary substances such as sugars (mannitol, sucrose, lactose, glucose, sucrose, trehalose, preferably 20-100 mg/ml) or amino acids such as glycine or arginine as well as antioxidants such as EDTA, citrate, polyethylene glycol (1-10 % by weight), detergents, preferably nonionic detergents (preferably 0.005 - 1 % by weight) such as polysorbates (Tween® 20 or Tween® 80) or polyoxyethylenes, anti-inflammatory ingredients, local anaesthetics, antibiotics and/or stabilizers such as lipids, fatty acids and glycerol.

In a further preferred embodiment, a pharmaceutical composition of the hedgehog protein according to the invention together with suramin is preferred and this can be used advantageously.

The pharmaceutical compositions according to claim 1 can contain additional pharmaceutical auxiliary substances.

In a preferred embodiment the pharmaceutical composition contains hedgehog protein at a concentration of 0.1 - 100 mg/ml.

In a preferred embodiment the pharmaceutical composition additionally contains a pharmaceutically acceptable buffer which is biocompatible preferably in a range between pH 4 and pH 10, particularly preferably in a range between pH 6 and 8, in particular at a pH value of ca. pH 7. The pH value of the pharmaceutical composition should be expediently more than pH 4 in order to prevent a denaturation and detachment of the zinc complexed in the hedgehog protein. The concentration of the buffer is preferably 1-500 mmol/l, in particular 5-150 mmol/l and particularly preferably 10-100 mmol/l. In a suitable embodiment 20 mmol/l potassium phosphate buffer, pH 7.2 or 100 mmol/l arginine chloride pH 7.2 is used as the buffer.

The following examples, publications and figures further elucidate the invention, the protective scope of which results from the patent claims. The described methods are to be understood as examples which still describe the subject matter of the invention even after modifications.

### Description of the Figures:

- **Figure 1:**: In vitro release of shh from a collagen matrix.
- **Figure 2:**: In vitro release of shh from calcium alginate capsules.
- **Figure 3:**: In vitro release of shh from hyaluronic acid gels.
- **Figure 4:**: In vitro release of shh from an alginate / collagen matrix.

### Examples

### Example 1

### Production of an alginate gel containing hh protein

An aliquot of hh protein solution (1 mg/ml shh solution in 50 mg/ml sucrose, 50 mM potassium phosphate, pH 7.2) is stirred with an Na-alginate stock solution (Pronova, Biopolymer, NO) (in water, greater than 0.1 %) in such a way that a gelatinous alginate protein mixture is formed. This gel can either be used directly as an injectable matrix or further processed into calcium alginate capsules or is stored as a lyophilisate.

### Example 2

### Production of a collagen mixture containing hh protein (comparative example)

100 µl of a hh solution (1 mg/ml hh) in
a) 20 mM potassium phosphate, pH 7.4 or
b) in 50 mM sodium acetate, pH 4.5 or
c) in 0.1 % trifluoroacetic acid, pH 2
is added dropwise onto collagen sponges (Helistat, Integra Life Sciences, USA) with a size of 10 x 10 x 3 mm. The loaded carriers are then frozen (-70°C), lyophilized and analysed.

For this the loaded sponges are incubated at 37°C in a suitable volume in buffer (10 mmol/l potassium phosphate, 150 mmol/l NaCl, pH 7.2). The amount of released hh is determined by means of RP-HPLC.

### Example 3

### 3.1 Production of Ca-alginate capsules

The alginate gel described in example 1 which contains hh protein is added dropwise to a CaCl₂ solution (about 1.5 %) while stirring continuously. Ca-alginate complexes form spontaneously which contain the protein. The size of the capsules that are formed depends on the size of the drops and can be varied as desired. After a 5 to 10 minute incubation in the CaCl₂ solution, the capsules are filtered and washed in buffer (20 mmol/l potassium phosphate, pH 7.2). These capsules can either be used directly as implants or be processed further by lyophilization.

### 3.2 Lyophilization

The Ca-alginate capsules are frozen at -70°C and subsequently lyophilized. The lyophilization enables a stable storage of the capsules and additionally facilitates the implantation since the capsules are easier to handle in the dry state.

### Example 4

### In vitro release of Shh

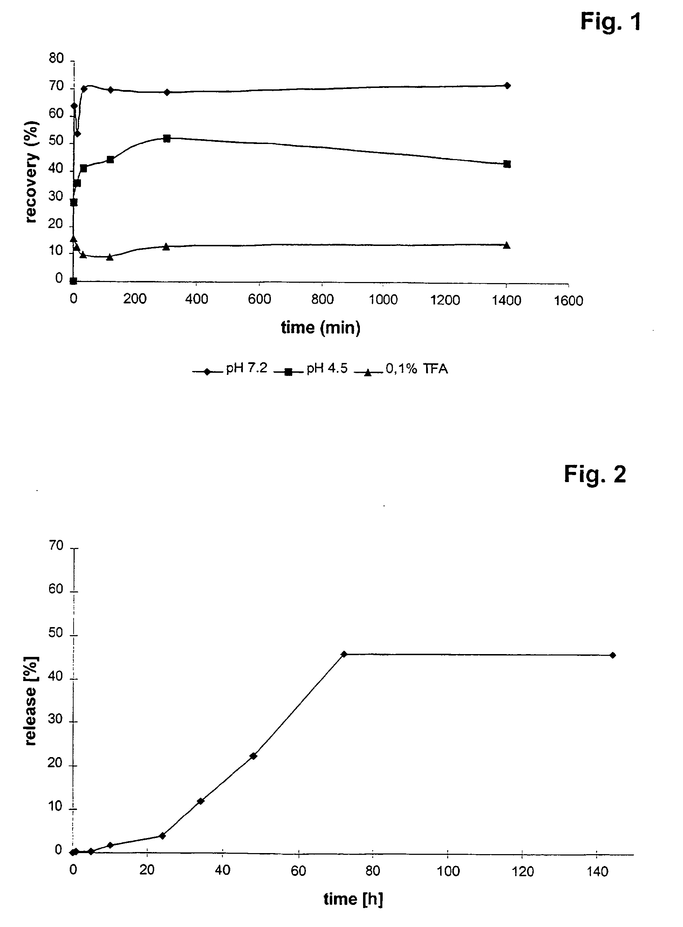

The analysis of the in vitro release kinetics shows that Shh is released in a delayed manner from Ca-alginate capsules over a period of at least 70 h (Fig. 2) whereas shh from a collagen matrix is released within a few, and up to ca. 20, minutes (Fig. 1). Lyophilized alginate spheres containing shh were incubated at 37°C in 10 mM potassium phosphate, 150 mM NaCl, pH 7.2 (Rotatherm). After 5 min, 1h, 5h, 10h, 1d, 34h, 2d, 3d and 6d samples are taken and analysed for their shh content by means of SDS polyacrylamide electrophoresis (Fig. 2). The cumulative amount of shh released from the alginate capsules is plotted against time.

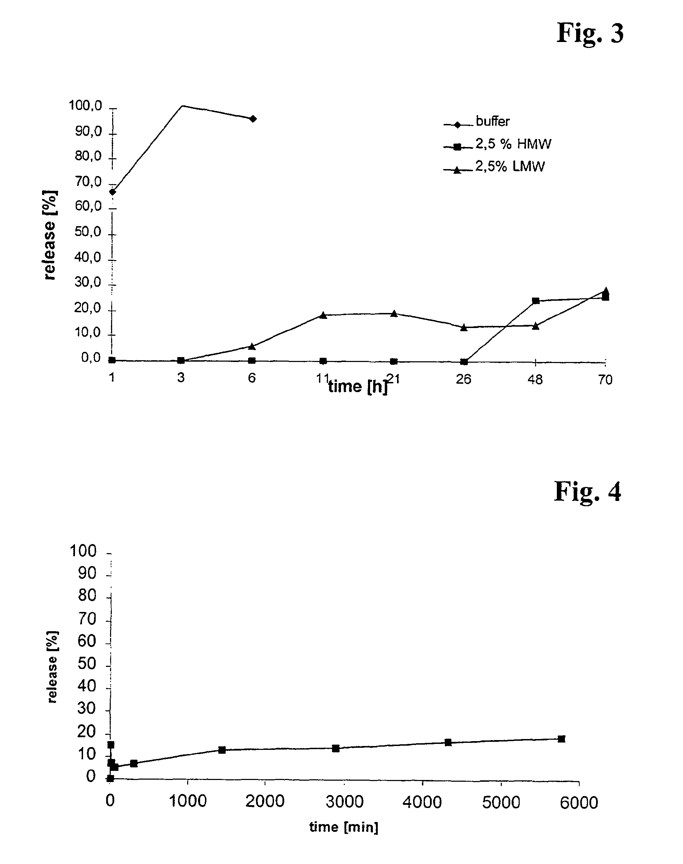

Examination of the release kinetics of shh from 2.5 % hyaluronic acid gels using hyaluronic acid types with a low molecular weight (LMW; molecular weight ca. 1.3 x 10⁶ Da) or with a high molecular weight (HMW; molecular weight ca. 4 x 10⁶ Da) is shown in Figure 3. For this hyaluronic acid gels loaded with shh are filled into dialysis tubes (separation size 300,000 Da) and the tubes are incubated in PBS at 37°C. At the stated times samples of the release medium were taken and analysed for their shh content by means of reversed phase HPLC. It is clear that a portion of the loaded hedgehog protein is released in a delayed manner. Another portion of the protein remains bound to the hyaluronic acid and could be released in vivo by degradation of the hyaluronic acid.

Examination of the release kinetics of shh from a collagen-alginate matrix (Fibracol^{™}, cf. U.S. Patent 4,614,794) is shown in Figure 4. For this a Fibracol sponge (1 x 1 x 0.3 cm) was loaded with 0.2 mg of hshh in PBS. The loaded sponges were frozen (-70°C), lyophilized and incubated in an appropriate volume of PBS at 37°C. At the stated times samples of the release medium were taken and analyzed for their shh content by means of reversed phase HPLC. It is clear that only about 10 to 20% of the loaded hedgehog proteins are released into the medium and that a major portion remains bound to the collagen-alginate matrix. This portion could be released in vivo by degradation of the matrix.

### List of References

Asahina, J., Exp. Cell. Res. 222 (1996) 38-47
Bitgood, M.J. et al., Curr. Biol. 6 (1996) 296
Chiang, C., et al., Nature 83 (1996) 407
Crey, C.J. et al., Biotechnol. Bioeng. 31 (1988) 607-612
Downs, E.C. et al., J. Cellular Physiology 152 (1992) 422-429
Fietz, M. et al., Development (Suppl) (1994) 43-51
Haque, Z. et al., J. Agric. Food Chem. 30 (1982) 481
Hynes, M. et al., Neuron 15 (1995) 35-44
Karablis et al., Genes and Development 8 (1994) 277-289
Kikuchi, A. et al., J. Controlled Release 47 (1997) 21-29
Kinto et al., FEBS Letters, 404 (1997) 319-323
Lai, C.J. et al., Development 121 (1995) 2349
Lopez-Martinez et al., Curr. Biol. 5 (1995) 791-796
Marti et al., Nature 375 (1995) 322-325
Miao et al., J. Neurosci. 17 (1997) 5891-5899
Nakamura, T. et al., Biochem. Biophys. Res. Comm. 237 (1997) 465-469
Perrimon, N., Cell 80 (1995) 517-520
Porter, J.A. et al., Science 274 (1996) 255-259
Robinson, C.J. et al., Trends in Biotechnology 14 (1996) 451-452
Smith, J.C., Cell 76 (1994) 193-196
Stott et al., J. Cell Sci. 110 (1997) 2691-2701
U.S. Patent 4,614,794
Vortkamp, A. et al., Science 273 (1996) 613
WO 90/08551
Yang et al., Development 124 (1997) 4393-4404

## Claims

1. Pharmaceutical composition for delayed release of a hedgehog protein in a pharmacologically effective dose over a period of at least 14 hours which is **characterized in that** the hedgehog protein is provided at a concentration of 0.1-100 mg/ml and is bound to a hydrophilic carrier which is biocompatible, wherein the carrier is a polymer which
- binds the hedgehog protein as a negatively-charged carrier as a result of ionic interactions,
- does not denature the hedgehog protein when it binds to the carrier,
- contains at least 0.1 to 2 negatively-charged residues per monomer under neutral conditions,
- contains the charge in the form of acidic groups,
- has an average molecular weight of at least 50,000 Da,
- and contains no agarose, and
wherein the pharmaceutical composition is buffered to maintain a pH in the range between 4 and 10.

2. Pharmaceutical composition of claim 1, wherein the hedgehog protein is provided at a concentration of 3mg/ml.

3. Pharmaceutical composition as claimed in claim 1, wherein the carrier is composed of an organic hydrophilic carrier matrix or of an inorganic insoluble phosphate.

4. Pharmaceutical composition as claimed in claims 1-3, wherein the carrier is composed of an anionic polysaccharide or an inorganic insoluble phosphate.

5. Pharmaceutical composition as claimed in claims 1-4, wherein the hydrophilic carrier is hyaluronic acid.

6. Pharmaceutical composition as claimed in claims 1-5, wherein the composition contains arginine or argininium ions.

7. Process for the production of a pharmaceutical composition for delayed release of a hedgehog protein in a pharmacologically effective dose over a period of at least 14 hours which contains a hedgehog protein at a concentration of 0.1-100 mg/ml and which is bound to a hydrophilic carrier that is biocompatible wherein the carrier is a polymer which
- binds the hedgehog protein as a negatively-charged carrier as a result of ionic interactions,
- does not denature the hedgehog protein when it binds to the carrier,
- contains at least 0.1 to 2 negatively-charged residues per monomer under neutral conditions,
- contains the charge in the form of acidic groups,
- has an average molecular weight of at least 50,000 Da,
- and contains no agarose,
wherein a hedgehog protein in a pharmaceutically effective amount is used as an essential component of this pharmaceutical composition, and wherein the pharmaceutical composition is buffered to maintain a pH in the range between 4 and 10.

8. Process as claimed in claim 7, wherein a hedgehog protein is used at a concentration of 0.1-100 mg/ml.

9. Process as claimed in claims 7-8, wherein the hydrophilic carrier is hyaluronic acid.

10. Process as claimed in claims 7-9, wherein the binding of the hedgehog protein to the hydrophilic carrier is effected by incubation at a pH of 4.5 or greater.

11. Use of the pharmaceutical composition of any of claims 1-5 in the manufacture of a medicament for the delayed release of a hedgehog protein in the human body, wherein the hedgehog protein is applied locally.

12. Use as claimed in claim 11, wherein the hedgehog protein is applied at a concentration of 0.1-100 mg/ml.

## Patentansprüche

1. Pharmazeutische Zusammensetzung für die verzögerte Freigabe eines Hedgehog-Proteins in einer pharmakologisch wirksamen Dosis während eines Zeitraums von mindestens 14 Stunden, **dadurch gekennzeichnet, daß** das Hedgehog-Protein in einer Konzentration von 0,1-100 mg/ml vorliegt und an einen hydrophilen Träger gebunden ist, der biokompatibel ist, wobei der Träger ein Polymer ist, welches
- das Hedgehog-Protein als einen negativ geladenen Träger als Ergebnis ionischer Wechselwirkungen bindet,
- das Hedgehog-Protein nicht denaturiert, wenn sich dieses an den Träger bindet,
- unter neutralen Bedingungen mindestens 0,1 bis 2 negativ geladene Reste pro Monomer enthält,
- die Ladung in Form von sauren Gruppen enthält,
- ein Durchschnittsmolekulargewicht von mindestens 50.000 Da aufweist sowie
- keine Agarose enthält, und
wobei die pharmazeutische zusammensetzung zur Aufrechterhaltung eines pH-Werts im Bereich zwischen 4 und 10 gepuffert ist.

2. Pharmazeutische Zusammensetzung nach Anspruch 1, wobei das Hedgehog-Protein in einer Konzentration von 3 mg/ml vorliegt.

3. Pharmazeutische Zusammensetzung nach Anspruch 1, worin der Träger aus einer organischen hydrophilen Trägermatrix oder einem anorganischen unlöslichen Phosphat besteht.

4. Pharmazeutische Zusammensetzung nach einem der Ansprüche 1 bis 3, worin der Träger aus einem anionischen Polysaccharid oder einem anorganischen unlöslichen Phosphat besteht.

5. Pharmazeutische Zusammensetzung nach den Ansprüchen 1 bis 4, worin der hydrophile Träger Hyaluronsäure ist.

6. Pharmazeutische Zusammensetzung nach den Ansprüchen 1 bis 5, worin die Zusammensetzung Arginin oder Argininiumionen enthält.

7. Verfahren zum Herstellen einer pharmazeutischen Zusammensetzung für eine verzögerte Freigabe eines Hedgehog-Proteins in einer pharmakologisch wirksamen Dosis während eines zeitraums von mindestens 14 Stunden, wobei die Zusammensetzung ein Hedgehog-Protein in einer Konzentration von 0,1-100 mg/ml enthält und an einen hydrophilen Träger gebunden ist, der biokompatibel ist, wobei der Träger ein Polymer darstellt, welches
- das Hedgehog-Protein als einen negativ geladenen Träger als Ergebnis ionischer Wechselwirkungen bindet,
- das Hedgehog-Protein nicht denaturiert, wenn sich dieses an den Träger bindet,
- unter neutralen Bedingungen mindestens 0,1 bis 2 negativ geladene Reste pro Monomer enthält,
- die Ladung in Form von sauren Gruppen enthält,
- ein Durchschnittsmolekulargewicht von mindestens 50.000 Da aufweist sowie
- keine Agarose enthält, und
wobei als wesentliche Komponente dieser pharmazeutischen Zusammensetzung ein Hedgehog-Protein in einer pharmazeutisch wirksamen Menge verwendet wird sowie die pharmazeutische Zusammensetzung zum Aufrechterhalten eines pH-Werts im Bereich zwischen 4 und 10 gepuffert ist.

8. Verfahren nach Anspruch 7, worin ein Hedgehog-Protein in einer Konzentration von 0,1-100 mg/ml verwendet wird.

9. Verfahren nach den Ansprüchen 7 bis 8, worin der hydrophile Träger Hyaluronsäure ist.

10. Verfahren nach den Ansprüchen 7 bis 9, worin das Binden des Hedgehog-Proteins an den hydrophilen Träger durch Inkubation bei einem pH-Wert von 4,5 oder höher bewirkt wird.

11. Verwendung der pharmazeutischen Zusammensetzungen nach einem der Ansprüche 1 bis 5 bei der Herstellung eines Arzneimittels für die verzögerte Freigabe eines Hedgehog-Proteins im menschlichen Körper, wobei das Hedgehog-Protein lokal angewandt wird.

12. Verwendung nach Anspruch 11, worin das Hedgehog-Protein in einer Konzentration von 0,1-100 mg/ml angewandt wird.

## Revendications

1. Composition pharmaceutique pour la libération retardée d'une protéine Hedgehog à une dose efficace du point de vue pharmacologique pendant une période d'au moins 14 heures, qui est **caractérisée en ce que** la protéine Hedgehog est fournie à une concentration de 0,1 à 100 mg/ml et est liée à un support hydrophile qui est biocompatible, dans laquelle le support est un polymère qui
- se lie à la protéine Hedgehog en tant que support chargé négativement à la suite d'interactions ioniques,
- ne dénature pas la protéine Hedgehog lorsqu'elle se lie au support,
- contient au moins 0,1 à 2 résidus chargés négativement par monomère dans des conditions neutres,
- contient la charge sous la forme de groupes acides,
- a un poids moléculaire moyen d'au moins 50.000 Da,
- et ne contient pas d'agarose, et
où la composition pharmaceutique est tamponnée de façon à conserver un pH compris dans la gamme de 4 à 10.

2. Composition pharmaceutique selon la revendication 1, dans laquelle la protéine Hedgehog est fournie à une concentration de 3 mg/ml.

3. Composition pharmaceutique selon la revendication 1, dans laquelle le support est composé d'une matrice de support hydrophile organique ou d'un phosphate insoluble inorganique.

4. Composition pharmaceutique selon les revendications 1 à 3, dans laquelle le support est composé d'un polysaccharide anionique ou d'un phosphate insoluble inorganique.

5. Composition pharmaceutique selon les revendications 1 à 4, dans laquelle le support hydrophile est l'acide hyaluronique.

6. Composition pharmaceutique selon les revendications 1 à 5, dans laquelle la composition contient de l'arginine ou des ions argininium.

7. Procédé de production d'une composition pharmaceutique pour la libération retardée d'une protéine Hedgehog à une dose efficace du point de vue pharmacologique pendant une période d'au moins 14 heures qui contient une protéine Hedgehog à une concentration de 0,1 à 100 mg/ml, laquelle est liée à un support hydrophile qui est biocompatible, dans laquelle le support est un polymère qui
- se lie à la protéine Hedgehog en tant que support chargé négativement à la suite d'interactions ioniques,
- ne dénature pas la protéine Hedgehog lorsqu'elle se lie au support,
- contient au moins 0,1 à 2 résidus chargés négativement par monomère dans des conditions neutres,
- contient la charge sous la forme de groupes acides,
- a un poids moléculaire moyen d'au moins 50.000 Da,
- et ne contient pas d'agarose,
dans lequel une protéine Hedgehog en une quantité efficace du point de vue pharmaceutique est utilisée en tant que composant essentiel de cette composition pharmaceutique, et dans lequel la composition pharmaceutique est tamponnée de façon à conserver un pH compris dans la gamme de 4 à 10.

8. Procédé selon la revendication 7, dans lequel une protéine Hedgehog est utilisée à une concentration de 0,1 à 100 mg/ml.

9. Procédé selon les revendications 7 et 8, dans lequel le support hydrophile est l'acide hyaluronique.

10. Procédé selon les revendications 7 à 9, dans lequel la liaison de la protéine Hedgehog au support hydrophile est effectuée par incubation à un pH de 4,5 ou plus.

11. Utilisation de la composition pharmaceutique selon l'une quelconque des revendications 1 à 5 dans la fabrication d'un médicament destiné à la libération retardée d'une protéine Hedgehog dans le corps humain, dans laquelle la protéine Hedgehog est appliquée localement.

12. Utilisation selon la revendication 11, dans laquelle la protéine Hedgehog est appliquée à une concentration de 0,1 à 100 mg/ml.
